# EUROPEAN PATENT APPLICATION

(11) **EP 1 698 267 A1**
(43) Date of publication of application: **06.09.2006**
(21) Application number: 04807084.1
(22) Date of filing: 15.12.2004
(51) Int. Cl.: A61B 1/00, A61J 3/07

(54) **CAPSULE DEVICE FOR MEDICAL USE**

(30) Priority: 25.12.2003 JP 2003431383
(71) Applicant: Olympus Corporation, Tokyo 151-0072 (JP)
(72) Inventor: Kojima, Kazuaki Olympus Int. Prop. Serv. Co., Ltd., Hashioji-shi Tokyo 192-8512 (JP)
(74) Representative: Brandl, Ferdinand Anton
(86) International application number: PCT/JP2004/018725
(87) International publication number: WO 2005/063111

(57) **Abstract**

With an object to provide a medical capsule apparatus which has various types of medical drugs depending on applications and can contribute to the reduction in management costs, the medical capsule apparatus put into the body cavity is therefore constituted such as to comprise at least a main body unit having a function for observing the body cavity and a medical drug unit having a function for accommodating and discharging a medical drug, the main body unit and the medical drug unit being freely connected and separated.

## Description

### Technical Field

The present invention relates to a medical capsule apparatus, and more particularly, to a medical capsule apparatus having observing means that observes the state of the body cavity in a substantially-capsule-shaped casing as a whole, with which a desired portion in the body cavity is observed and a medical drug or the like is sprayed.

### Background Art

One conventional endoscope apparatus comprises a tube inserting portion having an image pick-up device at the distal end thereof, an operating portion continuously-arranged to the inserting portion, and various devices such as an image processing device, a display unit, and a light source device which are connected to the operating portion. In the examination of the body cavity or the like, the inserting portion is inserted into the body cavity from the mouth of an examinee and a desired portion of the body cavity is observed. The conventional endoscope apparatus described as above is put into practical use and is widely used. However, the conventional endoscope apparatus described as above has a limit of the length of the inserting portion that is inserted in the body cavity. Consequently, the range of the observation or examination is limited.

Recently, a compact endoscope for accommodating image pick-up means (observing means) including an image pick-up optical system, illuminating means, communication means, power receiving means and a power supply, for example, in a capsule casing is variously suggested. That is, a medical capsule apparatus called a capsule endoscope is variously suggested.

Further, Japanese Unexamined Patent Application Publication No. 2-19140 discloses another conventional medical capsule apparatus in which a medical drug is accommodated in a capsule, an examinee swallows a capsule apparatus into the body cavity, then, the medical drug accommodated in the capsule is arbitrarily discharged out of the capsule by driving means using the electric control operation.

In the conventional medical capsule apparatus disclosed in Japanese Unexamined Patent Application Publication No. 2-19140, a mechanochemical substance which expands by receiving a voltage application or a change of a pH level and then increases in volume is used, thereby discharging the medical drug accommodated in the capsule out of the capsule.

Further, in the observation of the body cavity using the one conventional medical capsule device having the observing means, it is excessively convenient to perform predetermined treatment such as an operation for spraying the medical drug to a found lesion portion in the body cavity of the examinee. The medical drug sprayed in this case may be a curing medical drug or a marking agent for specifying the lesion portion.

Thus, the medical capsule apparatus observes the body cavity and simultaneously executes treatment on the desired portion. Further, in the detailed examination using a normal endoscope after the examination using the medical capsule apparatus, the operation for re-finding the lesion portion as a target of the endoscope examination is easy and the treatment is performed fast without fail.

### Disclosure of Invention

### Problems to be Solved by the Invention

In view of the form of the capsule main body of the conventional medical capsule endoscopes, observing means for observing the body cavity and a mechanism for accommodating and discharging the medical drug are integrally structured.

However, with the above-mentioned structure, the medical capsule apparatus must be provided corresponding to every kind of the used medical drug. Therefore, the structure has a problem that a user must prepare medical capsule apparatuses varied depending on any medical drugs which may be used.

Also in the medical capsule apparatus disclosed by the Japanese Unexamined Patent Application Publication No. 2-19140, for example, any means for observing a body cavity has not been taken into consideration, so that there is a problem that it is difficult to reliably spray a medical drug or the like for a desired portion in the body cavity.

In view of this, the present invention has been achieved, and an object of the present invention is to provide a medical capsule apparatus having observing means for observing a body cavity and a mechanism for accommodating and discharging a medical drug or the like, wherein a main body unit having the observing means for observing a body cavity and a medical drug unit having the mechanism for accommodating and discharging a medical drug or the like are separately provided, both of which being constituted so as to be easily connected and separated, thereby making it possible to have various types of medical drugs depending on applications and also contribute to the reduction in management costs.

### Means for Solving the Problem

In order to meet the object as mentioned above, the medical capsule apparatus in accordance with the present invention is characterized in that a medical capsule apparatus put into the body cavity comprises at least a main body unit having a function for observing the body cavity, and a medical drug unit having a function for accommodating and discharging a medical drug, wherein the main body unit and the medical drug unit are freely connected and separated.

### Effects of the Invention

In accordance with the present invention, it is possible to provide a medical capsule apparatus having observing means for observing a body cavity and a mechanism for accommodating and discharging a medical drug or the like, wherein a main body unit having the observing means for observing a body cavity and a medical drug unit having the mechanism for accommodating and discharging a medical drug or the like are separately provided, both of which being constituted so as to be easily connected and separated, thereby making it possible to have various types of medical drugs depending on applications and also contribute to the reduction in management costs.

### Brief Description of the Drawings

Fig. 1 is a block diagram schematically showing the internal structure of a medical capsule apparatus according to a first embodiment of the present invention;
Fig. 2 is a conceptual diagram showing a separating state of a main body unit and a medical drug unit in the medical capsule apparatus according to the first embodiment;
Fig. 3 is a conceptual diagram showing a solving state of a soluble film when the main body unit is connected to the medical drug unit in the medical capsule apparatus shown in Fig, 1 and the main body unit and the medical drug unit are put in the body cavity;
Fig. 4 is a conceptual diagram showing a state of discharging a medical drug from the medical drug unit by operating a magnetic-force generating portion of the main body unit in the medical capsule apparatus shown in Fig. 1;
Fig. 5 is a diagram schematically showing the arrangement of internal members in a medical capsule apparatus according to a second embodiment of the present invention; and
Fig. 6 is a cross-sectional view along VI-VI-line in Fig. 5.

### Best Mode for Carrying Out the Invention

Hereinbelow, a description is given of embodiments of the present invention with reference to the drawings.

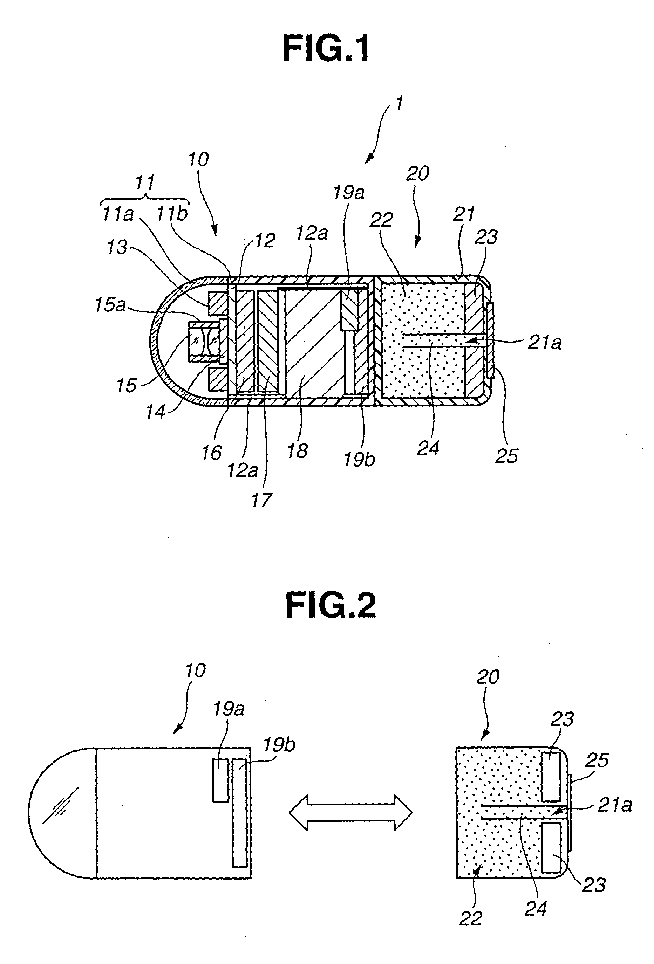

Fig. 1 is a block diagram schematically showing the internal structure of a medical capsule apparatus according to a first embodiment of the present invention. Referring to Fig. 1, the cross section of the medical capsule apparatus is shown, thus showing the internal structure.

Referring to Fig. 1, a medical capsule apparatus (hereinafter, referred to as a capsule apparatus) 1 according to the first embodiment comprises: a main body unit 10 having the arrangement of various components such as observing means for observing the body cavity in a casing; and a medical drug unit 20 having a mechanism which accommodates and discharges a medical drug.

Fig. 1 shows a connecting state of the main body unit 10 and the medical drug unit 20. In the connecting state shown in Fig. 1, the external shape of the capsule apparatus 1 is substantially capsule-shaped as a whole.

Separately from the main body unit 10 and the medical drug unit 20, the capsule apparatus 1 comprises an external control device (not shown) comprising control means which externally controls the main body unit 10.

The main body unit 10 comprises a main-body-unit exterior case 11 serving an exterior member for watertightly sealing the main body unit 10 and various components arranged in the main-body-unit exterior case 11. For example, the main body unit 10 comprises in the main-body-unit exterior case 11: an image pick-up unit serving as observing means comprising an illumination 13 serving as illuminating means and a light source, optical lenses 15 serving as an optical system having a plurality of optical devices, a lens barrel 15a which holds the optical lenses 15, a solid-state image pick-up device 14 which generates an image signal of a subject, and a signal processing portion 16 serving as signal processing means which processes an output signal from the solid-state image pick-up device 14; a circuit substrate 12 having the signal processing portion 16, the illumination 13, an electric circuit for controlling the driving operation of the signal processing portion 16 and the illumination 13, and a control circuit which systematically controls electric circuits in the capsule apparatus 1; a radio receiving/sending portion 17 serving as communication means which receives an output signal from the signal processing portion 16 and receives/sends the signal between the main body unit 10 and the external control device; a battery 18 which supplies power to the electric components in the capsule apparatus 1; medical drug discharge control means having a magnetic-force generating portion 19b which controls a signal for generating the magnetic force and a magnetic-force control portion 19a which controls the driving operation of the magnetic-force generating portion 19b, the magnetic force generated from the magnetic-force generating portion 19b, and the discharge operation of a medical drug 22; a flexible printed circuit (FPC) board 12a which electrically connects the circuit substrate 12 to the radio receiving/sending portion 17 or the battery 18 and the magnetic-force generating portion 19b to the magnetic-force control portion 19a; and the like.

The main-body-unit exterior case 11 contains a rigid member such as resin, and comprises: a transparent window portion 11a which covers and protects the front portion of the main body unit 10 in the capsule apparatus 1 and through which illuminated beams outputted from the illumination 13 or beams incident on the optical lenses 15 are passed; and a main body portion 11b which comprises a main portion of the main-body-unit exterior case 11 with internal components and externally covers and protects the internal components.

Among the components of the image pick-up unit serving as the observing means, the optical lenses 15 comprise a plurality of lenses as mentioned above. The optical lenses 15 are held by the lens barrel 15a.

On the rear side along the optical axis of the optical lenses 15 held by the lens barrel 15a, the circuit substrate 12 is arranged at a predetermined position perpendicularly to the optical axis. The solid-state image pick-up device 14 is mounted on the circuit substrate 12, having the light receiving surface thereof in the front-surface direction. The solid-state image pick-up device 14 comprises an image pick-up device such as a CCD or a CMOS which receives an optical image of the subject that passes through the optical lenses 15 and is formed and which performs photoelectrical converting processing.

Mounted on the circuit substrate 12 is an electric circuit comprising a plurality of electric parts which drive the solid-state image pick-up device 14 and perform predetermined signal processing. Thus, the optical image of the subject formed by the optical lenses 15 is formed onto the light receiving surface of the solid-state image pick-up device 14, predetermined photoelectrical converting processing is performed, and a predetermined image signal is generated.

The illumination 13 is a light source such as an LED (light emitting device) or an organic EL which illuminates the subject such as the digestive organ in the body cavity. The illumination 13 is mounted on the circuit substrate 12 so as to illuminate the subject on the front side of the capsule apparatus 1.

The illumination 13 illuminates the subject and the illuminating beams are reflected by the subject. Then, the reflected beams are condensed by the optical lenses 15. After the condensed beams pass through the optical lenses 15, the optical image of the subject is formed onto the light receiving surface of the solid-state image pick-up device 14.

The solid-state image pick-up device 14 receives the optical image of the subject formed by the optical lenses 15, performs the signal processing such as the predetermined photoelectrical converting processing, and generates an electric signal (image signal) corresponding to the optical image of the subject.

The image signal outputted from the solid-state image pick-up device 14 is subjected to various signal processing (including image signal processing and communication processing) in the signal processing portion 16. Thereafter, the image signal is outputted to the radio receiving/sending portion 17.

As mentioned above, the medical-drug discharge control means comprises the magnetic-force generating portion 19b and the magnetic-force control portion 19a, and forms a mechanism for externally discharging the medical drug 22 accommodated in a medical drug unit 20 which will be described later. In this case, the magnetic-force control portion 19a receives a predetermined radio signal from a radio sending device externally-arranged to the body cavity, and controls the magnetic force of the magnetic-force generating portion 19b based on the received signal (which will be described in detail later).

The medical drug unit 20 comprises: a medical-drug-unit exterior case 21 which contains a rigid member such as resin and has a space for filling the medical drug 22 therein; the medical drug 22 which is filled in the medical-drug-unit exterior case 21; a magnetic-force absorbing portion 23 which absorbs the magnetic force; a spray nozzle 24 which externally discharges and sprays the medical drug 22 filled in the medical-drug-unit exterior case 21; a soluble film 25 serving as spray nozzle opening/closing means which is at one end of the spray nozzle 24 and is arranged to close and watertightly seal a hole portion 21a of the medical-drug-unit exterior case 21.

The used medical drug 22 is a medical drug for curing, a marking agent which specifies the lesion portion, or the like.

The magnetic-force absorbing portion 23 contains a plate member which absorbs magnetic force. Further, the magnetic-force absorbing portion 23 is arranged so as to be moved in parallel with the absorbing direction of the magnetic-force generating portion 19b in the medical drug unit 20 upon generating the magnetic force from the magnetic-force generating portion 19b by the magnetic-force control portion 19a of the main body unit 10.

The spray nozzle 24 comprises a hollow tube member which is communicated with the hole portion 21a formed substantially in the center of one side surface of the medical-drug-unit exterior case 21 and which is arranged toward the inside of the medical-drug-unit exterior case 21. The spray nozzle 24 is arranged to externally discharge the medical drug 22 filled in the medical-drug-unit exterior case 21.

That is, the magnetic-force control portion 19a of the main body unit 10 generates the magnetic force from the magnetic-force generating portion 19b and then the magnetic-force absorbing portion 23 of the medical drug unit 20 is moved in parallel with the direction for absorbing the magnetic-force absorbing portion 23 of the medical drug unit 20 to the magnetic-force generating portion 19b. Then, the medical drug 22 filled in the medical drug unit 20 is pressed. Therefore, the medical drug 22 is externally discharged out of the medical-drug-unit exterior case 21 via the spray nozzle 24.

The soluble film 25 contains a thin-film member made of a soluble component such as gelatin in the body cavity. The hole portion 21a of the medical-drug-unit exterior case 21 is watertightly closed by using the soluble film 25. Thus, the soluble film 25 prevents the leakage of the medical drug 22 from the medical-drug-unit exterior case 21 and the invasion of molds and the like in the medical-drug-unit exterior case 21.

Upon using the capsule apparatus 1, that is, under the environment in the body cavity, the soluble film 25 is solved. Therefore, when the capsule apparatus 1 reaches a desired portion in the body cavity and then the medical drug 22 is discharged, the closed state of the hole portion 21a of the medical-drug-unit exterior case 21 is reset and the medical drug 22 is easily discharged.

The main body unit 10 and the medical drug unit 20 with the above-mentioned structure are connected by mechanical connecting means such as a screwing tool or a screw stop tool. Although not shown, a male screw is formed on the outer circumferential surface side at one end (on the arranging side of the magnetic-force generating portion 19b) of the main-body-unit exterior case 11 in the main body unit 10. A female screw is formed near another end (on the opposite side of the formed side of the hole portion 21a) of the medical-drug-unit exterior case 21 in the medical drug unit 20. Further, the main body unit 10 and the medical drug unit 20 are connected by the screw connection or the like.

Hereinbelow, a description is given of the operation of the capsule apparatus 1 with the above-mentioned structure according to the first embodiment.

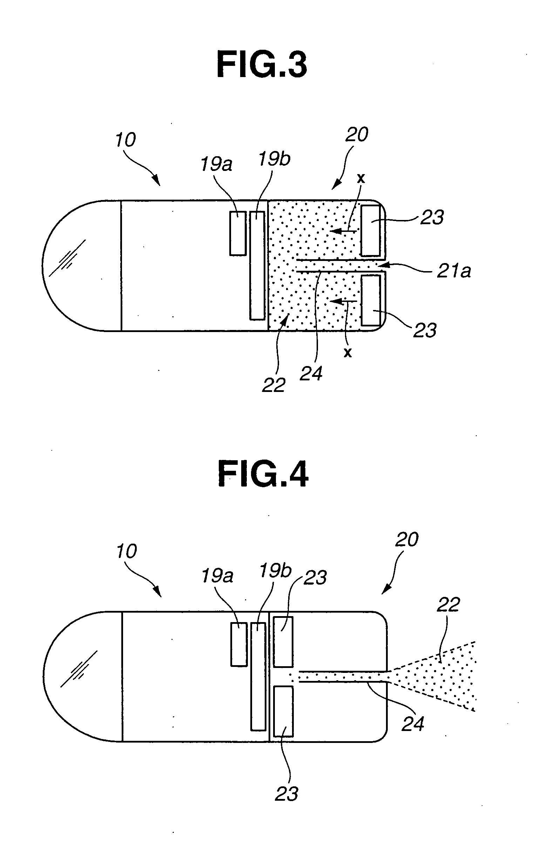

Fig. 2 is a conceptual diagram showing the separating state of the main body unit 10 and the medical drug unit 20 in the capsule apparatus 1. Fig. 3 is a conceptual diagram showing the state of solving the soluble film 25 when the main body unit 10 is connected to the medical drug unit 20 in the medical capsule apparatus 1 shown in Fig. 1 and the main body unit 10 and the medical drug unit 20 are put in the body cavity. Fig. 4 is a conceptual diagram showing the state of discharging the medical drug 22 from the medical drug unit 20 by operating the magnetic-force generating portion 19b of the main body unit 10 in the medical capsule apparatus 1 shown in Fig. 1. The structure of the capsule apparatus 1 is simply shown in Figs. 2 to 4, and the internal structure is partly omitted.

Referring to Fig. 2, in the capsule apparatus 1 before use, the main body unit 10 and the medical drug unit 20 are separated from each other. In using the capsule apparatus 1, the medical drug unit 20 corresponding to the application is selected from a plurality of medical drug units 20 containing different kinds of medical drug, and the selected medical drug unit 20 is mechanically connected to the main body unit 10. Then, the state shown in Fig. 1 is obtained.

The examinee swallows the capsule apparatus 1 in which the main body unit 10 and the medical drug unit 20 are connected. Thus, the capsule apparatus 1 is moved in the body cavity of the examinee by the peristaltic motion. In this case, the image pick-up unit operates on the main body unit 10 side and the state of the body cavity is observed.

That is, while the capsule apparatus 1 is moved in the body cavity, the illumination 13 illuminates the body cavity. In this case, the optical lenses 15 form the optical image of the body cavity onto the light receiving surface of the solid-state image pick-up device 14. The solid-state image pick-up device 14 receives the optical image, photoelectrically converts the image, generates an electric signal, and outputs the generated signal to the signal processing portion 16. The signal processing portion 16 performs predetermined signal processing and then outputs the processed signal to the radio receiving/sending portion 17. The radio receiving/sending portion 17 communicates the input image signal by predetermined radio waves and sends the signal to the external control device (not shown). The external control device which receives the sent signal performs predetermined signal processing and outputs the processed signal to a monitor device (not shown) or a recording device. As a consequence, the image of the body cavity is displayed on the monitor device and the recording device records the image signal indicating the image.

After the examinee swallows the capsule apparatus 1 and the capsule apparatus 1 is put in the body cavity, the soluble film 25 arranged to the medical drug unit 20 of the capsule apparatus 1 starts to gradually be solved under the environment of the body cavity and then reaches the state shown in Fig. 3. In this state, the hole portion 21a of the medical-drug-unit exterior case 21 in the medical drug unit 20 is opened.

An operator of the capsule apparatus 1 observes the image of the body cavity displayed on the monitor device and simultaneously waits until the capsule apparatus 1 reaches a desired portion (lesion portion) in the body cavity. When the capsule apparatus 1 reaches the desired portion in the body cavity, the operator executes a predetermined operation of the external control device, and sends a predetermined control signal to the main body unit 10. The control signal generated in this case is an instructing signal for controlling the driving operation of the magnetic-force generating portion 19b via the magnetic-force control portion 19a and for generating predetermined magnetic force. The instructing signal is communicated to the magnetic-force control portion 19a via the radio receiving/sending portion 17. Then, the magnetic-force control portion 19a controls the driving operation of the magnetic-force generating portion 19b, and thus generates the predetermined magnetic force from the magnetic-force generating portion 19b. The generated magnetic force acts on the magnetic-force absorbing portion 23 of the medical drug unit 20 and the magnetic-force absorbing portion 23 starts to be moved in the direction (X direction shown in Fig. 3) for absorption to the magnetic-force generating portion 19b. In accordance therewith, referring to Fig. 4, the medical drug 22 filled in the medical-drug-unit exterior case 21 is externally sprayed from the hole portion 21a via the spray nozzle 24. As a consequence, the medical drug 22 is adhered to a desired portion of the lesion portion in the body cavity of the examinee.

After that, the capsule apparatus 1 continuously moves by the peristaltic motion in the body cavity and then is naturally discharged out of the body.

As mentioned above, according to the first embodiment, the main body unit 10 including the image pick-up unit for observing the body cavity and the medical drug unit 20 for accommodating the medical drug 22 therein and arbitrarily discharging it are separately structured, and are connected by easy means. Thus, the medical drug unit 20 is selected in accordance with the application and the selected medical drug unit 20 is just connected to the main body unit 10, thereby conveniently structuring the capsule apparatus 1 suitable to the case.

The medical drug unit 20 is just varied depending on the kinds of the medical drug 22 filled therein. The number of the main body units 10 comprising a relatively expensive component need not be prepared the same number as the medical drug units 20, thereby reducing the management costs.

Further, the main body unit 10 can be reused and therefore this contributes to the further reduction in management costs.

The structure of the medical drug unit 20 is simplified, and the medical drug unit 20 is manufactured as a single unit. Therefore, a device manufacturer of the main body unit 10 can be different from that of the medical drug unit 20. Since many medical drug manufacturers share the manufacture of the medical drug unit 20, this contributes to the reduction in manufacturing costs.

According to the first embodiment, the main body unit 10 in the capsule apparatus 1 includes the battery 18, and the battery 18 supplies the power to the internal circuits in the main body unit 10. However, the present invention is not limited to this.

For example, among the internal components of the main body unit 10 according to the first embodiment, in place of the radio receiving/sending portion 17 and the battery 18, a radio power feed portion for receiving and sending the control signal and supplying the power by external radio means may be arranged. With the above-mentioned structure, the same advantages as those according to the first embodiment are obtained.

Positional information in the body cavity on the lesion portion is grasped by the diagnosis result obtained in advance by another means. In view of this, the main body unit 10 in the capsule apparatus 1 according to the first embodiment may comprise predetermined position detecting means which detects the position of the capsule apparatus 1 inserted in the body cavity, in place of the observing means such as the image pick-up unit that is relatively expensive. The above-mentioned position detecting means is used together with the positional information on the lesion portion that is grasped in advance and then the same advantages as those according to the first embodiment are obtained while realizing the reduction in total costs.

Next, according to a second embodiment, a description is given of a case in which the main body unit side of the capsule apparatus comprises in-body information detecting means (in-body sensing unit) which detects the state in the body, in place of the observing means.

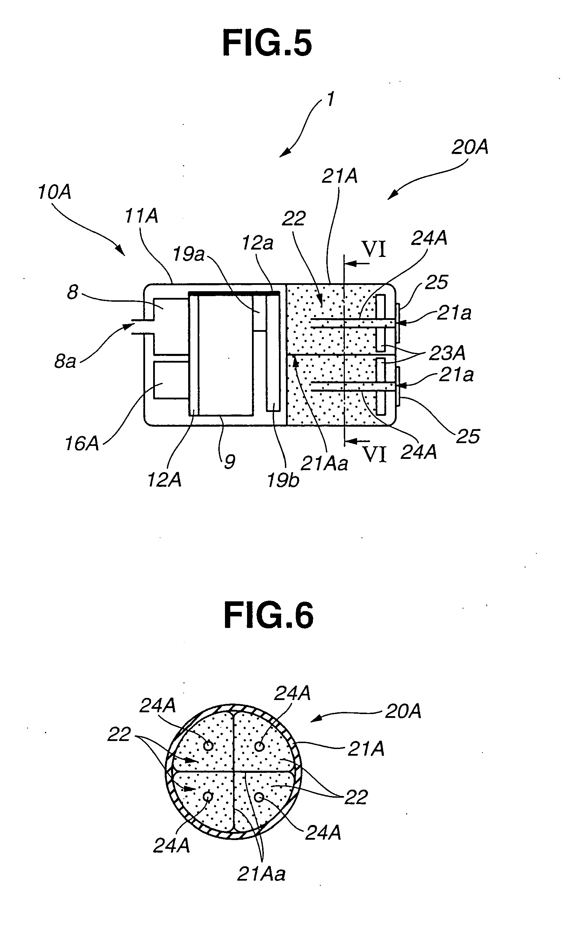

Fig. 5 is a schematic diagram conceptually showing the arrangement of internal components of a medical capsule apparatus according to the second embodiment of the present invention. Fig. 6 is a cross-sectional view along VI-VI-line in Fig. 5.

Referring to Fig. 5, a capsule apparatus 1A according to the second embodiment comprises: a main body unit 10A having the arrangement of various components such as an in-body sensing unit serving as the in-body information detecting means for detecting a desired portion in the body cavity; and a medical drug unit 20A (also refer to Fig. 6) having a mechanism for accommodating a plurality of medical drugs and discharging the medical drugs.

Incidentally, Fig. 5 shows the connecting state of the main body unit 10A and the medical drug unit 20A. In the connecting state shown in Fig. 5, the outer shape is substantially capsule-shaped as a whole.

In the capsule apparatus 1A, similarly to the first embodiment, independently of the main body unit 10A and medical drug unit 20A, the capsule apparatus 1A further comprises an external control device (not shown) comprising control means for externally controlling the main body unit 10A.

The main body unit 10A comprises: a main-body-unit exterior case 11A serving as an exterior member for watertightly sealing the inside; and various components arranged in the main-body-unit exterior case 11A. For example the main body unit 10A comprises in the main-body-unit exterior case 11A: an in-body sending unit, serving as the in-body information detecting means comprising a sensor 8 functioning as observing means for observing the state in the body cavity by detecting the state of the body cavity and a signal processing portion 16A serving as signal processing means for processing an output signal from the sensor 8; a circuit substrate 12A having a control circuit for entirely controlling the signal processing portion 16A, the sensor 8, an electric circuit for controlling the driving operation of the signal processing portion 16A and the sensor 8, and an internal electric circuit of the capsule apparatus 1A; a radio power feed portion 9, serving as communication means which receives a signal outputted form the signal processing portion 16A and receives/sends the signal between the main body unit 10A and the external control device, which feeds, by predetermined radio means, power from an external power supply and which supplies the power to an electric component in the capsule apparatus 1A; medical drug discharge control means comprising the magnetic-force generating portion 19b for controlling a signal for generating the magnetic force and the magnetic-force control portion 19a for controlling the driving operation of the magnetic-force generating portion 19b; the flexible printed circuit (FPC) board 12a which electrically connects the circuit substrate 12A to the radio power feed portion 9, the magnetic-force control portion 19a, and the magnetic-force generating portion 19b; and the like.

The main-body-unit exterior case 11A contains a rigid member such as resin, and the internal component in the main body unit 10A of the capsule apparatus 1A is covered and is protected.

The sensor 8 has a communicating portion 8a which is partly communicated with the outside of the main-body-unit exterior case 11A. Thus, the state of the body cavity serving as the external state of the main-body-unit exterior case 11A is detected. The communicating portion 8a is unnecessary depending on the type of the sensor 8. For example, a detecting sensor using light does not need the communicating portion 8a, and may have a window portion containing transparent resin at a predetermined portion of the main-body-unit exterior case 11A corresponding to the detecting device of the sensor 8.

The sensor 8 for detecting and observing the state of the body cavity (also referred to as in-body information) may be a pH sensor, a temperature sensor, a pressure sensor, or a detecting sensor of the blood or tumor marker.

The radio power feed portion 9 comprises the same functions as those of the radio receiving/sending portion according to the first embodiment, that is, a portion having a function for receiving an output signal from the sensor 8, sending the received signal to the external control device, and receiving the control signal from the external control device, and a portion having a function for supplying the power from the external power to a battery portion for feeding and charging the power from the external power supply by predetermined radio means, an electric member of the capsule apparatus 1A, the sensor 8, the signal processing portion 16A, and the medical-drug discharge control means.

The magnetic-force control portion 19a and the magnetic-force generating portion 19b forming the medical-drug discharge control means are components having the same functions as those according to the first embodiment.

The medical drug unit 20A according to the second embodiment comprises: a medical-drug-unit exterior case 21A which contains a rigid member such as resin and has a space for filling the medical drug 22 therein; the medical drug 22 which is filled in the medical-drug-unit exterior case 21A; a magnetic-force absorbing portion 23A which absorbs the magnetic force; a spray nozzle 24A which externally discharges and sprays the medical drug 22 filled in the medical-drug-unit exterior case 21A; a soluble film 25 which is at one end of the spray nozzle 24A and is arranged to close and watertightly seal the hole portion 21a of the medical-drug-unit exterior case 21A.

In the internal space of the medical-drug-unit exterior case 21A, a plurality of spaces are formed by partitions 21Aa (refer to Fig. 6). In the individual spaces, different medical drugs 22 or the same type of the medical drug 22 is filled. Further, in the individual spaces, the magnetic-force absorbing portion 23A and the spray nozzle 24A are arranged. Furthermore, the holes 21a are pierced in the individual spaces. The hole portions 21a are closed by the soluble film 25. Thus, in the single medical drug unit 20A, a plurality of kinds of medical drugs 22 are simultaneously filled.

The magnetic-force absorbing portions 23A arbitrarily operate by controlling the driving operation of the magnetic-force generating portion 19b by the magnetic-force control portion 19a of the main body unit 10A. Therefore, a plurality of medical drugs 22 are discharged at varied timings.

Other structures are the same as those according to the first embodiment. Referring to Fig. 5, the components having the same numeral as those according to the first embodiment have the same functions.

The operation of the capsule apparatus 1A with the above-mentioned structure according to the second embodiment is substantially the same as that according to the first embodiment. However, since the in-body sensing unit is arranged as the in-body information detecting means in place of the image pick-up unit as the observing means according to the first embodiment, the operation of the in-body sensing unit will mainly be described.

Similarly to the first embodiment, the capsule apparatus 1A is formed by combining and connecting the medical drug unit 20A in which the medical drug 22 is filled in accordance with the application and the main body unit 10A having the sensor 8 in accordance with the application.

The examinee swallows the capsule apparatus 1A and then the soluble film 25 is solved in the body cavity similarly to the first embodiment. Then, the hole portions 21a of the medical-drug-unit exterior case 21A are opened.

Upon moving the capsule apparatus 1A in the body cavity, the capsule apparatus 1A is moved while the sensor 8 detects the state of the body cavity via the communicating portion 8a. An output of the sensor 8 is transmitted to the radio power feed portion 9 via the signal processing portion 16A and is sent to the external control device via the radio power feed portion 9. Consequently, the operator continuously recognizes the state of the body cavity.

The capsule apparatus 1A is monitored while the detecting result of the sensor 8 is observed. When the capsule apparatus 1A reaches a desired portion, the operator performs predetermined operation of the external control device and a predetermined control signal is sent to the magnetic-force control portion 19a via the radio receiving/sending portion of the radio power feed portion 9 of the main body unit 10A.

Alternatively, when the capsule apparatus 1A reaches a desired portion, a predetermined operation is automatically performed by the signal from the sensing unit, and a predetermined control signal is sent to the magnetic-force control portion 19a.

Thus, the magnetic-force control portion 19a generates the magnetic force from a predetermined portion of the magnetic-force generating portion 19b. The magnetic-force absorbing portion 23A corresponding to the space in which the desired medical drug 22 is filled is operated, thereby externally spraying the medical drug 22 from the hole portions 21a via the spray nozzle 24A. Then, the medical drug 22 is adhered to a desired portion of the lesion portion in the body cavity of the examinee. The same operation is performed in accordance with the types of the medical drugs 22 filled if necessary.

After that, the capsule apparatus 1A continuously moves by the peristaltic motion in the body cavity, and is naturally discharged out of the body without operations.

As mentioned above, according to the second embodiment, the same advantages as those according to the first embodiment are obtained.

Further, according to the second embodiment, the internal structure of the medical drug unit 20A is improved so as to simultaneously fill the medical drug unit 20A with a plurality of types of medical drugs 22 individually. Further, the medical drugs 22 are individually discharged out at an arbitrary time. Thus, a single capsule apparatus 1A corresponds to a plurality of medical drugs 22 and to the more complicated curing.

## Claims

1. A medical capsule apparatus put into the body cavity comprising at least:
a main body unit having a function for observing the body cavity; and
a medical-drug unit having a function for accommodating and discharging a medical drug,
wherein the main body unit and the medical drug unit are constituted so as to be freely connected and separated.

2. A medical capsule apparatus according to Claim 1, wherein the main body unit comprises at least:
observing means for observing the body cavity; and
medical-drug discharge control means for receiving a signal from the observing means and controlling the discharge operation of the medical drug.

3. A medical capsule apparatus according to Claim 1, wherein the main body unit comprises at least:
observing means for observing the body cavity;
communication means for externally receiving/sending a signal; and
medical-drug discharge control means for receiving a signal from the communication means and controlling the discharge operation of the medical drug.

4. A medical capsule apparatus according to Claim 2 or 3, wherein the observing means comprises at least:
illuminating means for irradiating a subject;
a solid-state image pick-up device;
an optical system for forming an optical image indicating the subject onto a light receiving surface of the solid-state image pick-up device; and
signal processing means for processing an output signal from the solid-state image pick-up device.

5. A medical capsule apparatus according to Claim 2 or 3, wherein the observing means is an in-body sensing unit comprising at least a sensor for sensing in-body information and signal processing means for processing a signal from the sensor.

6. A medical capsule apparatus according to Claim 2 or 3, wherein the medical-drug discharge control means comprises at least:
a magnetic-force generating portion for generating magnetic force; and
a magnetic-force control portion for controlling the magnetic force of the magnetic-force generating portion and controlling the discharge operation of the medical drug.

7. A medical capsule apparatus according to any one of Claims 1 to 6, wherein the medical-drug unit comprises at least:
a medical drug;
a magnetic-force absorbing portion which is arranged at the facing position of the magnetic-force generating portion in the medical-drug discharge control means via the medical drug and which is absorbed by the magnetic-force generating portion;
a spray nozzle for spraying the medical drug; and
spray nozzle opening/closing means for opening/closing the spray nozzle.

8. A medical capsule apparatus according to Claim 7, wherein the spray nozzle opening/closing means comprises a soluble film which is solved in the body.
